# EUROPEAN PATENT SPECIFICATION

(11) **EP 1 940 459 B1**
(45) Date of publication and mention of the grant of the patent: **09.04.2014**
(21) Application number: 06802990.9
(22) Date of filing: 05.09.2006
(51) Int. Cl.: A61M 37/00, A61K 9/00

(54) **SOLID SOLUTION PERFORATOR CONTAINING DRUG PARTICLE AND/OR DRUG-ADSORBED PARTICLES**
FESTER LÖSUNGSPERFORATOR MIT EINEM ARZNEITEILCHEN UND/ODER ARZNEIADSORBIERTEN TEILCHEN
PERFORATEUR A SOLUTION SOLIDE CONTENANT DES PARTICULES DE MEDICAMENT ET/OU DES PARTICULES A ADSORPTION DE MEDICAMENT

(30) Priority: 06.09.2005 US 714469 P
(43) Date of publication of application: 09.07.2008
(73) Proprietor: Theraject, Inc., Fremont, CA 94538 (US); Kwon, Sung-Yun, Femont, CA 94536 (US)
(72) Inventor: KWON, Sung-Yun, CA 94536 (US)
(74) Representative: Feldmeier, Jürgen
(86) International application number: PCT/US2006/034606
(87) International publication number: WO 2007/030477

(56) References cited:
- WO-A1-2007/012114
- WO-A2-02/064193
- WO-A2-03/002069
- US-B1- 6 256 533
- US-B1- 6 334 856
- US-B1- 6 511 463
- US-B1- 6 555 157
- US-B2- 6 713 081

## Description

### TECHNICAL FIELD

This invention relates to controlled delivery of one or more drugs to, and diagnosis of fluids in, a patient's body.

### BACKGROUND OF THE INVENTION

Many new biopharmaceutical drugs, including proteins, peptides, nucleotide and DNA constituents and genes, have been developed for better and more efficient treatment for disease and illness. Especially due to recent advances in molecular biology and biotechnology, biotechnology-derived therapeutic proteins, such as recombinant human insulin, growth hormone and erythropoeitin, to name only a few, are now available. However, a major limitation in using these new drugs is lack of an efficient drug delivery system; a drug must be transported across one or more biological barriers in the body at rates and in amounts that are therapeutically effective.

Most drugs are orally administered. However, some drugs, especially protein and peptide drugs, cannot be effectively adsorbed in this manner because of severe degradation in the gastrointestinal tract, poor absorption in intestinal membrane and/or first pass breakdown by the liver.

Another administration technique is parental injection, using standard syringes or catheters. Needle injection provokes needle phobia, substantial pain, local damage to the skin in many patients. Withdrawal of body fluids, such as blood, for diagnostic purposes provokes similar discomforts. Further, needle injection is not ideal for continuous delivery of a drug, or for continuous diagnosis.

Another drug delivery technique is transdermal delivery, which usually relies on diffusion of a drug across the skin. This method is not broadly applicable because of the poor skin permeability of many drugs. The outermost layer of skin, stratum corneum, represents a major barrier to transdermal drug penetration. Once a drug reaches the dermal depth (below the epidermal layer), the drug diffuses rapidly to deep tissue layers and other parts of the system via blood circulation.

In an attempt to improve the rate of protein drug delivery through the skin, chemical enhancers, iontophoresis, electroporation, ultrasound, and heat elements have been used to supplement drug delivery. However, these techniques are not suitable for some types of drugs and often fail to provide a therapeutic level of delivery. These techniques sometimes result in undesirable skin reactions and/or are impractical for continuous controlled drug delivery over a period of hours or days.

Other attempts, such as particle or liquid injection, have been made to design alternative techniques to transfer drugs transdermally. A main advantage of those techniques is absence of needle use and reduction of incidence of contamination. However, liquid injection frequently causes some pain and/or sub-dermal hemorrhage. One technique, ballistic particle injection, is hard to administer exactly and continuously and can cause microbleeding.

Others have used microneedles (less than 1mm in diameter) to effect percutaneous drug delivery. Microneedles have been used to deliver a drug through a lumen in the needles, to deliver a drug along the outside of the needle shafts, or as skin perforators for subsequent patch drug application. Silicon microneedles, for example, have been developed using fabrication procedures from the semiconductor industry. Examples are described in US 6334856 to Allen et al. (Jan. 2001), US 6256533 to Yuzhakov, et al. (July 2001), US 6312612 to Sherman, et al., (Nov. 2001), and US 6379324 to Gartstein, et al. (April 2002). Unfortunately, silicon needles are not dissolvable in the skin, and when broken during use can produce considerable irritation and even infection.

WO02/064193, WO03/002069 and WO2007012114 demonstrate microneedle compositions and their corresponding methods of manufacturing.

There remains a need for an approach that reduces or controls the skin barriers to permit controlled introduction of one, two or more drugs simultaneously or sequentially, and to provide prompt drug delivery with inexpensive fabricating and various patch designs including dissoluble microneedles.

### SUMMARY OF THE INVENTION

These needs are met by the invention, which applies mechanical penetration of the skin, using a dissoluble solid solution perforator ("SSPP") system containing particle drug or drug-adsorbed particles, and dissolves or undergoes biodegradation relatively quickly, such as within 1 minute to 24 hours, preferably within 5 minutes to 10 hours, such as within 10 minutes to 5 hours, or any time period within these ranges. An "SSPP device" optionally includes a reservoir of a second drug, contained in a patch, located adjacent to the perforator array and containing either the same drug as is contained in the SSPP system perforators or a different drug. By creating a drug transport channel or port in the skin, especially in the outermost layer, through use of an SSPP (system) perforator, the barrier properties of skin can be diminished or controlled for drug delivery and for providing access to body fluids to be monitored. Optionally, a patch includes a ring of adhesive that bonds with, and holds the SSPP against the patient's skin adjacent to the perforated region of the skin. The patch system is separately activated to deliver the second drug through the skin channels(s) formed by the SSPP perforator(s).

In contrast to conventional hollow needle technologies, the SSPP system includes a solid matrix of dissolvable (including meltable) material that holds one or more selected drug particles and/or drug-loaded particles and is formed into one or more perforators. The matrix can be composed of fast-dissolving and/or swelling materials. The solid solution can be a homogenous or a non-homogeneous phase, such as a suspension solution.

The drug suspension solid solution can consist of hydrophobic drug particles in a hydrophilic matrix. The drug can be, but is not limited to an organic molecule or a macromolecule such as a vaccine or protein drug. The drug can be adsorbed on an inert particle embedded in the dissoluble matrix. Drug adsorption to the inert particle can be achieved due to the high surface energy of the particle (for example, the surface area of aluminum hydroxide is 500m²/g) or by physical bonding such as by hydrophobic interaction and/or electrostatic interaction. A surface area of 510 m²/g is unusual for a crystalline material and approaches the surface area values reported for expandable clay minerals that range from 600 to 800 m²/g.

One of skill in the art can readily determine the amount of protein that can be adsorbed to a particular particle. Exemplary protein adsorption amounts on aluminum hydroxide are as follows: 1.6-3.1 mg bovine serum albumin/mg; 2.6 mg ovalbumin/mg; 1.9 mg a-lactalbumin/mg; 1.1 mg myoglobin/mg.

One particular advantage of using a drug suspension, a drug particle or a drug-loaded particle with the SSPP, is that the drug can be concentrated at the microneedle tip or surface by various fabrication methods and parameters, such as through centrifugation. By microneedle tip is meant the tapered end of the microneedle. Generally, drug will be concentrated in the bottom half to third of the microneedle, preferably in the bottom quarter or less of the portion of the microneedle that forms the pointed tip. The drug-loaded particle and tip-concentrated microneedle is especially beneficial for potent protein drug delivery, such as protein therapeutics and vaccines, because this design allows conservation of the drug and therefore provides an efficient and economical method for drug delivery.

Thus, in one embodiment, the invention is directed to a method of producing a microneedle with a selected drug concentrated in the tip or on the tip surface. The method comprises:
(a) providing a particulate component selected from the group consisting of a particulate drug, and an inert particle with a drug adsorbed thereto;
(b) combining the particulate component with a soluble matrix material to form a suspension solution comprising the particulate component;
(c) casting the suspension solution into a microneedle mold;
(d) centrifuging the cast microneedle mold under conditions that move the particulate component into the microneedle tip or tip surface; and
(e) drying and separating the cast microneedle from the mold.

In another embodiment, the invention is directed to a method of producing a microneedle with a selected drug concentrated in the tip or on the tip surface. The method comprises:
(a) combining a selected drug, a soluble matrix material and an inert particle in solution to form a suspension solution comprising the inert particle with the drug and matrix adsorbed thereto;
(b) casting the suspension solution into a microneedle mold;
(c) centrifuging the cast microneedle mold under conditions that move the drug-adsorbed inert particle into the microneedle tip or surface of the microneedle; and
(d) drying and separating the cast microneedle from the mold.

In yet another embodiment, the invention is directed to a method of producing a microneedle with a selected drug concentrated in the tip or on the tip surface. The method comprises:
(a) providing a particulate component selected from the group consisting of a dried particulate drug, and a dried inert particle with a drug adsorbed thereto;
(b) adding the particulate component into the tip portion of a microneedle mold;
(c) packing a powdered matrix onto the particulate component to fill the microneedle mold;
(d) applying a compressive force to the packed microneedle mold to solidify the microneedle; and
(e) drying and separating the cast microneedle from the mold.

In certain embodiments of all of the above methods, the drug is a vaccine and the inert particle is poly (lactic-co-glycolic acid) (PLGA) or aluminum hydroxide and aluminum phosphate (alum). In alternative embodiments, the drug is a protein.

In yet further embodiments, the matrix material is a hydrogel. In certain embodiments, the matrix material comprises sodium carboxymethyl cellulose. In additional embodiments, the matrix material further comprises vitamin C.

These and other embodiments of the subject invention will readily occur to those of skill in the art in view of the disclosure herein.

### BRIEF DESCRIPTION OF THE FIGURES

FIG. 1 is a schematic cross-section of a patient's skin.
FIG. 2 shows an exemplary fabricating procedure for a solid perforator containing
   particles with a suspension solution.
FIG. 3 is an exemplary fabricating procedure for compacting powder for a solid perforator.
FIGS. 4A-4E are schematic diagrams of the procedure for preparing a microneedle containing drug particles or drug-adsorbed particles.
FIGS. 5A-5B show ZnO₂ particles (FIG. 5A) and a particle-loaded microneedle (FIG. 5B).
FIG. 6 shows a cross-section of a representative drug patch system that includes a drug reservoir.

### DETAILED DESCRIPTION OF THE INVENTION

The practice of the present invention will employ, unless otherwise indicated, conventional methods of chemistry, biochemistry, pharmacology and drug delivery, within the skill of the art. Such techniques are explained fully in the literature..

All publications, patents and patent applications cited herein, whether *supra* or *infra,* are hereby incorporated by reference in their entirety.

It must be noted that, as used in this specification and the appended claims, the singular forms "a", "an" and "the" include plural referents unless the content clearly dictates otherwise. Thus, for example, reference to "a protein" includes a mixture of two or more polypeptides, and the like.

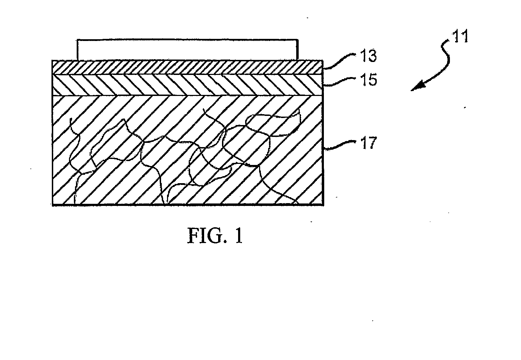

FIG. 1 is a cross-sectional view of the top layers of the skin 11, including a stratum corneum 13, an epidermal layer or epidermis 15 and a dermal layer or dermis 17. The outermost layer of skin, the stratum corneum 13, is a dead cell layer, usually between 10 and 20 microns (µm) thick. The stratum corneum 13 contains hydrophilic keratinocytes surrounded by a hydrophobic extracellular matrix of lipids, mainly ceramide. Due to the structural and compositional uniqueness, the stratum corneum 13 presents the greatest barrier to transdermal flux of drugs or other molecules into the body, and of body fluids and other analytes out of the body. The stratum corneum 13 is continuously renewed by shedding of corneum cells, with an average turnover time of 2-3 weeks.

Below the stratum corneum 13 is the viable epidermis or epidermal layer 15, which is between 50 and 100 µm thick. The epidermis contains no blood vessels and freely exchanges metabolites by diffusion to and from the dermis 17, located immediately below the epidermis 15. The dermis is between 1 and 3 mm thick and contains blood vessels, lymphatics, and nerves. Once a drug reaches the dermal layer, the drug will perfuse through system circulation.

The inert particles for drug adsorption can be ZnO₂, poly (lactic-co-glycolic acid) (PLGA) and other biopolymer particles, gold particles, alum, (aluminum hydroxide and aluminum phosphate), nanoparticles, calcium phosphate, other clay particles, such as but not limited to sodium bentonite, calcium bentonite, sodium cloisite, and kaolin. In certain embodiments, the particles are drug particles themselves that are precipitated from a super-saturated matrix. The particle drug may display different dissolution rates from the drug in the matrix. The drug dissolution rate can be effected by combining the drug particles and drug in the matrix.

Optionally, a drug concentration gradient can be made in the microneedle. In this embodiment, the suspension is concentrated in the microneedle by, for example, centrifugation, which moves the particles to the tip of the micromold, the movement depending on the rotating speed and density difference between the matrix and particles. The microneedle is then dried and separated from the mold and used as a patch component. This unique fabrication method can therefore be used to concentrate drug onto the tip and/or surface of the microneedle. The tip- concentrated microneedle is especially useful for delivery of vaccines or potent protein drugs. Drug concentrated on the tip can be more economical by allowing the use of less drug and can provide for enhanced delivery efficiency. The drug-loaded particles from the tip or surface of the microneedle can stay in tissue for sustained drug delivery even after the patch is removed. The drug adsorbed on the solid particle is significantly more stable than the free form of drug.

Other fabrication methods for use with the present invention include compaction and compression. Where a powder form of drug-adsorbed particles and matrix powders are used for the SSPP material, a mixed powder is spread over the mold. Depending upon the chemical and physical properties of the powder, appropriate heating of the powder may be applied to melt or spray various viscous materials or solvent into the mold. The powder may be compacted by pressure and/or application of heating, with or without use of binding agents. When SSP perforators have been formed into an array, the SSPP array is cooled, separated from the mold, and incorporated into an SSP system.

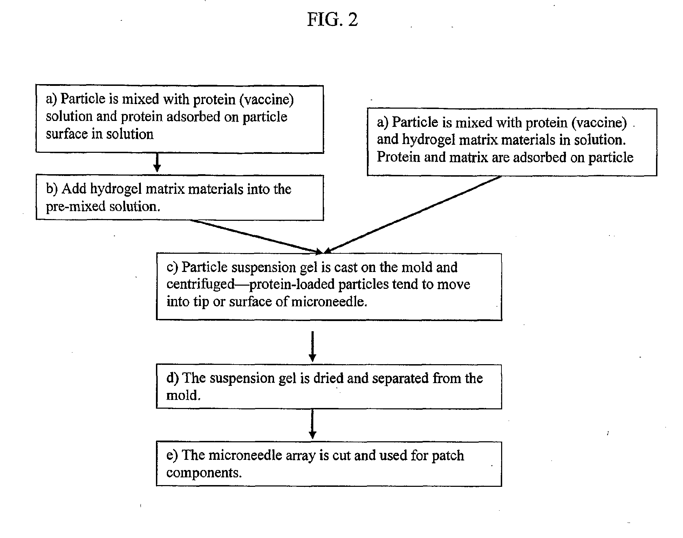

FIG. 2 shows a representative fabrication method for a drug-loaded particle microneedle. The vaccine or protein is mixed with the particle and adsorbed on the particle surface. The soluble matrix materials are added into the solution and the suspension gel solution with drug-adsorbed particles are cast on the mold and centrifuged. In the centrifuge process for filling the solution into the microneedle, the drug-adsorbed particle tends to move to the tip or surface of the needle because of the higher density of the particles as compared to the matrix gel (specific gravity of most particulates in this application is 1.5-2.5 and for a metal particle can be 15-25). Because of this difference, more particles are located in the tip or surface region. The high concentration on the tip is useful, effective and cost-saving for delivery of potent, expensive drugs. Once centrifuged, the microneedle is dried, separated from the mold and cut for a component of a patch. An alternative method, is to mix the drugs and soluble matrix materials first, then add the particles. In this way, the amount of drug adsorbed on the particle surface can be controlled. By changing the separation time from the mold, the final dimension of the microneedle can be adjusted. In the mold process, silicone gel can be used to make mold replicates. This provides for efficient mass production.

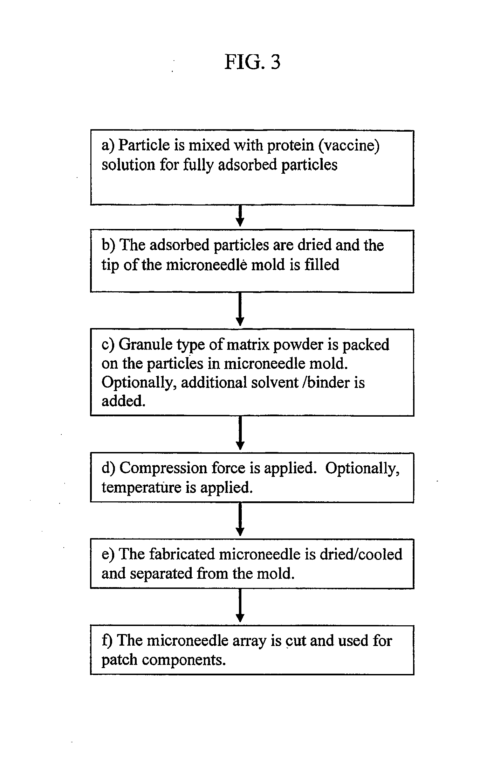

FIG. 3 depicts an alternative method for fabricating a drug-loaded particle microneedle. In this embodiment, the vaccine or protein is mixed with the particle of interest and adsorbed on the particulate surface. The microneedle mold is then filled with a predetermined dose of the drug-adsorbed particles. In this process, a small particle size is preferred and a tapping process can be used to easily fill the mold. Preferably, the drug-adsorbed are applied to the tip portion of the microneedle mold. By tip portion is meant approximately the bottom half to third of the microneedle mold that tapers into a point. The matrix powder is then packed on to the mold, optionally with additional solvent or binder to solidify the microneedle. A compressive force is applied to solidify the microneedle. In this process, the temperature can be increased for effective solidification. When heating is applied, the temperature control is critical. The applied temperature and duration should be lower and shorter enough to avoid any degradation or chemical reactions between excipients. With this process, a drug concentration gradient can be built into the microneedle. The highly concentrated drug on the tip may be preferred for economical reasons, especially when potent, expensive drugs are used. The microneedle is dried, cooled and separated from the mold and cut for a component of the patch.

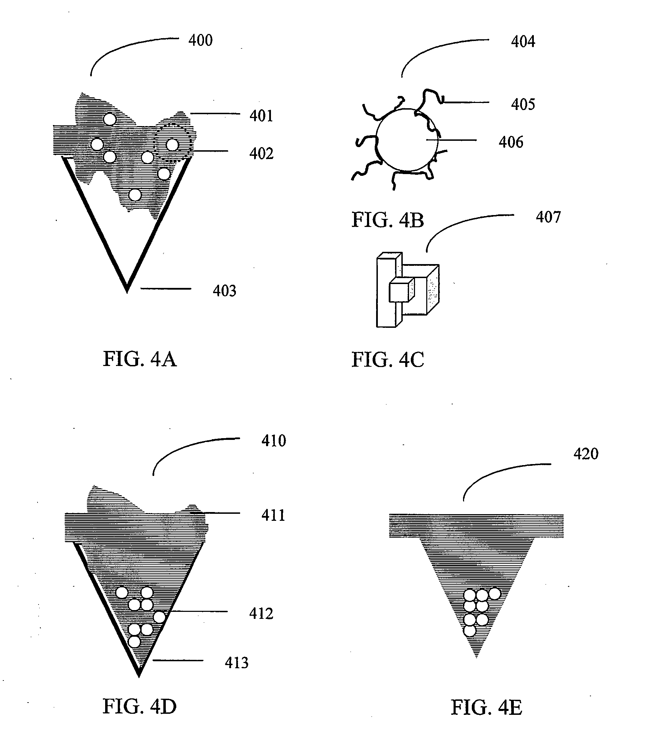

FIGS. 4A-4E show a fabricating method for a drug-loaded particle microneedle 400. The hydrogel matrix mixture 401 with drug-loaded particle 402 or drug precipitant are cast into the microneedle mold 403. The magnified images of drug-adsorbed particle and precipitant (assumed crystal) are shown as 404 in FIG. 4B and 407 in FIG. 4C. The drug-adsorbed particle, such as a large molecular weight protein or vaccine are shown as 405 and the particle is 406. A particle suspension gel under centrifugation is depicted at 410 in FIG. 4D. The mixture gel 411 fills the micromold 413 and drug-loaded particle 412 tends to move to the tip when centrifuged and is concentrated into the tip or surface of the microneedle FIG. 4D. After drying, the microneedle is separated from the mold 420 in FIG. 4D.

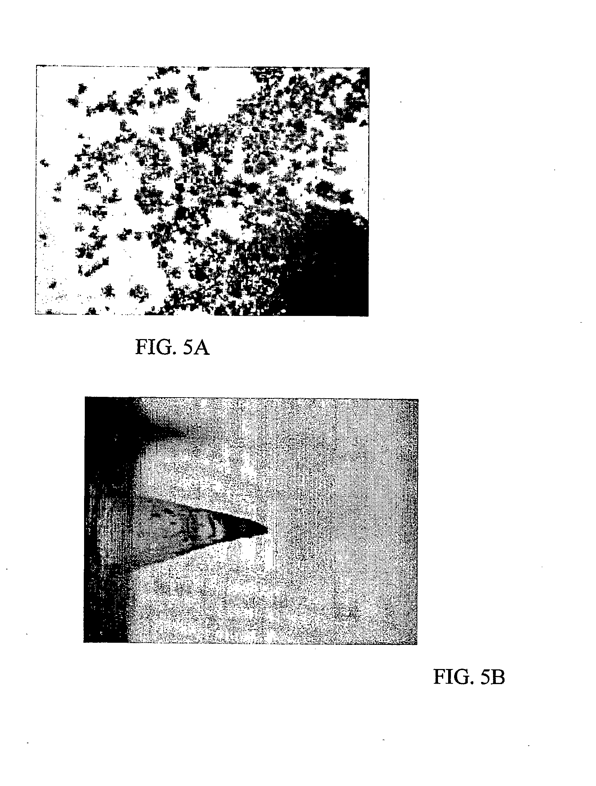

FIGS. 5A-5B are actual images of a ZnO₂ particle (FIG. 5A) and the particle-embedded in microneedle (FIG. 5B). In this example, the average particle size is about 1µm and microneedle length is 900 µm. In the centrifuge fabrication process, using 3500 rpm for 5 minutes, the ZnO₂ particle is well concentrated in the tip of microneedle because of the high density compared to the matrix materials.

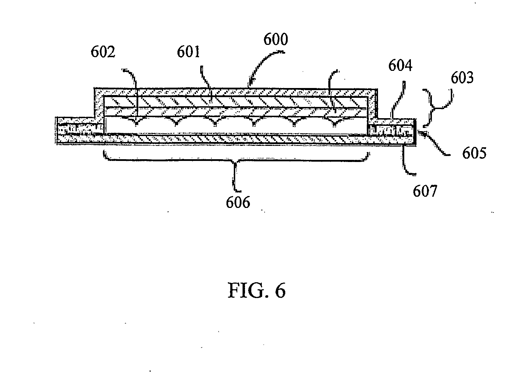

Optionally, a drug patch system 600, illustrated in FIG. 6, includes a drug reservoir 601, containing a second drug that may be the same as or different from the first drug, that is located above and adjacent to the SSPP perforator array 602 and that has an independently controlled reservoir drug delivery system 603. The drug patch system 600 preferably includes a backing film 604 that surrounds the drug reservoir 601 and includes an annular adhesive region 605 that surrounds and seals off the SSPP skin perforation region 606. A plastic release liner 607 is peeled off before skin perforation and protects an SSPP system until the liner is peeled off.

The SSPP perforators can have straight or tapered shafts or can be pyramids or wedges or blades. In a preferred embodiment, the outer diameter of an SSPP perforator is greatest at the base or second end, about 1-2000 µm, and the perforator outer diameter near the first end is preferably 1-100 µm. The length of an SSPP perforator is typically in a range 10-5000 µm, more preferably in a range 100-3000 µm. The average particle size can be 0.01 -100 µm, the particles having a broad size distribution. The skin is not a smooth, but rather a rugged surface and has different depths microscopically. In addition, the thickness of the stratum corneum and elasticity of the skin varies from person to person and from location to location on any given person's body. A desirable penetration depth has a range, rather than a single value, for effective drug delivery and relatively painless and bloodless penetration. Penetration depth of an SSPP perforator can affect pain as well as delivery efficiency. In certain embodiments, the perforator penetrates to a depth in the range of 10-1000 µm. In transdermal applications, the "penetrated depth" of the SSPP perforator is preferably less than 100 µm so that a perforator, inserted into the skin through the stratum corneum, does not penetrate past the epidermis. This is an optimal approach to avoid contacting nerves and blood vessels. In such applications, the actual length of the SSPP perforator can be longer because the basal layer associated with the SSPP system may not be fully inserted into the skin because of elasticity and rough surface of the skin.

Depending upon medical needs, perforator penetration to the dermis layer may be required in some applications. In these instances, use of an SSPP system can be a practical option in handling instant drug delivery situations. The penetrating portion of an SSPP perforator can be optimized by adjusting perforator variables (SSPP length, dimension, mechanical properties of basal or substrate layer as well as stroke and speed of insertion of an SSPP perforator), as well as accounting for target skin elasticity, skin hardness and surface roughness. The insertion speed can be increased with a microneedle injector operated by spring, gas, mechanical or electronic force.

The primary functions of an SSPP perforator are to pierce the stratum corneum, to provide prompt initiation of drug delivery from the matrix or drug-adsorbed particle and optionally to help keep the channels open for subsequent drug delivery or body fluid monitoring. As long as an SSPP perforator dissolves reasonably quickly and provides drug-loaded particles and is strong enough to pierce the stratum corneum, any biocompatible material can serve as an SSPP perforator.

In preparing an SSPP perforator, a mold is prepared using precision machining, micro-machining, or laser-based or electro-discharge machining. A silicone replica can be easily and inexpensively prepared from the mold with silicone curing. When the mold is prepared, a liquid solution, including the matrix material and including the selected drug(s) or drug-loaded particles, is cast in the mold and dried. Depending on the viscosity and other physical and chemical properties of the liquid solution, additional force such as centrifuge force or compression force may be needed to fill the mold. Elevated temperatures may optionally be used. To form a solid solution, the solvent is dried using any of various known methods, such as but not limited to air-drying, vacuum-drying or freeze-drying. Once a solid solution is formed, an SSPP perforator is separated from the mold and cut to an appropriate shape and size for patch component. For a description of representative shapes and sizes of such perforators, see, e.g., International Publication No. WO 2004/000389, published December 31, 2203, incorporated herein by reference in its entirety.

Suitable matrix materials for an SSPP perforator include polymers, including but not limited to sodium carboxymethyl cellulose (SCMC), polyvinylpyrolidone (PVP), polyethylene glycol (PEG), polyvinyl alcohol (PVA), polyethylene oxide (PEO), maltodextrin, polyacrylic acid, polystylene sulfonate, polypeptide, cellulose, hydroxypropyl cellulose (HPC), hydroxyethyl cellulose (HEC), hydroxypropyl methylcellulose (HPMC), dextrin, dextran, mono- and polysaccharide, polyalcohol, gelatin, gum arabic, alginate, chitosan cylcodextrin and other water dissolvable natural and synthetic polymers, or combinations of the above.

Carbohydrate derivatives, such as sugar derivatives (trehalose, glucose, maltose, lactose, maltulose, iso-maltulose, lactulose, fluctose, turanose, melitose, mannose, melezitose, dextran, maltotol, sorbitol, xylitol, inositol, palatinit and mannitol) can be used. Water-soluble ingredients, such as phosphate, nitrate and carboxylate glasses, magnesium chloride, potassium chloride and calcium chloride can be also used for a matrix material, alone or mixed with a matrix polymer.

The matrix can also include vitamin C or vitamin C derivatives. Vitamin C can diminish potential skin reactions. Additionally, vitamin C reduces viscosity of the matrix for a better centrifuge process.

As explained above, the inert particle materials for drug absorption can be any of various particles well known in the art, including but not limited to, ZnO₂, PLGA particles, other bioplastic particles, aluminum hydroxide, alum, alum phosphate, calcium phosphate, nanoparticles, clay particles, such as sodium bentonite, calcium bentonite, sodium cloisite, kaolin, hydroxyapatite, inert metal particles such as gold, titanium, and metal alloy particles. Additionally, any water-insoluble particles or precipitants in an aqueous matrix and any insoluble particles or precipitants in a nonaqueous matrix, can be used for drug adsorption and as delivery carriers. For example, alum or PLGA particulates are beneficial for vaccine formulation and delivery since these particles act as adjuvants to boost the immune response, stabilize the adsorbed drugs, as well as provide a depot effect for sustained desorption. Since particle size is small, providing a high surface energy, as well as hydrophobic, providing for hydrophobic bonding with the hydrophobic part of a protein drug, and optionally electrostatic bonding, the protein drug is easily adsorbed onto the particle surface and is not easily de-bound from the particle surface in the fabrication process. For diagnostic applications, a sensor protein or enzyme (for example glucose oxidase for glucose monitoring) can be adsorbed or immobilized on the particle or sensor particle Optionally, the surface properties can be modified by various techniques, such as silanization, plasma treatment, surface coating, polymer surface grafting etc., in order to control bonding with the protein drug.

In some cases, the particle can be a precipitated drug particle from a saturated matrix and the precipitants act as additional drug adsorbants or other drug adsorbants.

An SSPP patch system optionally includes a reservoir containing a liquid or gel form of the second drug and one or more perforators extending from at least a part of the reservoir's surface. The SSPP perforators associated with the patch system penetrate the stratum corneum of the skin to enhance percutaneous drug administration and to provide prompt drug delivery and/or prompt drug cut off. In the patch system, the SSPP perforators and the reservoir can be constructed as a single unit or as separate units.

An SSPP patch system is applied to the skin so that one or more SSPP perforators penetrate through the stratum corneum, into the epidermis or into the dermis depending on the application. In a preferred embodiment, drug-loaded particles in the dissolvable matrix microneedle tip or surface, dissolve into the epidermis or dermis. For vaccination, the vaccine-loaded or coated adjuvant particles can maximize vaccination. The vaccine molecules or antigen detach from the particle surface and diffuse or migrate into the epidermis, such as, for example, into Langerhans cells.

An SSPP system can transport therapeutic and/or prophylactic agents, including drugs and vaccines and other bioactive molecules, across skin and other tissues. An SSPP device permits drug delivery and access to body fluids across skin or other tissue barriers, with minimal damage, pain and/or irritation at the tissue. In drug delivery applications, an SSPP perforator is primarily composed of an active drug-loaded particle (or drug particle itself) and a dissolving solid matrix depending on a desired drug profile. The SSPP system acts as an immediate drug source and as a channel creator for subsequent drug delivery through skin. Depending on the application, an osmotically active or anti-irritant compound, such as vitamins, can have a beneficial effect. In diagnostic applications, the SSPP perforator can include or consist of sensor materials loaded or embedded particles that react to the presence of specific analytes.

In certain situations it is useful to have anti-virus and/or anti-bacterial protection in the basal layer to suppress infection. In order to vary or control the drug delivery rate, an external physical enhancement system, using iontophoresis, or sonophoresis, piezoelectric response, a heating element or a similar response, can be provided with the overlay layer.

Any drug or other bioactive agent can be delivered using the SSPP system with drug-loaded or coated particles. Delivered drugs can be proteins, peptides, nucleotides, DNA, genes, polysaccharides, and synthetic organic and inorganic compounds. Representative agents include, but are not limited to, anti-infectives, hormones, growth regulators, drugs regulating cardiac action or blood flow, and drugs for pain control. The drug can be for vaccination or local treatment or for regional or systemic therapy. The following are representative protein drugs and examples of useful doses per injection:
□-interferon 11-100 µg;
□-interferon for multiple sclerosis 22-44 µg;
Erythropoetin (EPO) for anemia 10-30 µg;
Follicle stimulating hormone (FSH) 5-30 µg;
parathyroid hormone (PTH) 20-40 µg;
Granulocyte Colony Stimulating Factor (G-CSF) 9-15 µg;
Granulocyte Macrophage Colony Stimulating Factor (GM-CSF) 250 µg;
Human chorionic gonadotropin 30-300 µg;
Leutinizing hormone 2-30 µg;
Salmon Calcitonin 25-50 µg;
Glucagon 1 mg;
GNRH antagonist 2 mg;
Insulin 0.75-1.5 mg;
Human Growth Hormone (GHD) 0.25-1.5 mg;
Human Growth Hormone (AIDS) 6 mg;
Testosterone 5-10 mg;
Lidocaine 2-5 percent;
Diclofenac Sodium 100-200 mg;
Oxybutynin 5-15 mg;
Ketoprofen 75-200 mg;
Alemdronate 10 mg;
Enalpril Maleate 10-40 mg;
Phenylpropanolamine HCl 75 mg;
Cromolyn sodium 3.2-10 mg;
Isotretinoin 0.5-2 mg/kg;
Oxytocin 1-2 unit/min/iv;
Paroxetine HCl 20 mg;
Flurbiprofen 100 mg;
Sertaline 50 mg;
Venlafaxine 75 mg;
Leuprolide 0.125-0.25 mg;
Risperidone 4-6 mg;
Galanthamine hydrobromide 16-24 mg;

Anticoagulant Enoxaprin, rheumatoid arthritis Etanercept, postoperative and chronic pain Fentanyl, low white blood cells from chemotherapy Filgrastin, anticoagulant Heparin, Parathyroid hormone (PTH), Somatropin, growth hormone Sumatriptan, migraine headaches Morphine Opiate anti-arthritis.

Many drugs can be delivered at a variety of therapeutic rates, controlled by varying a number of design factors including: dimensions of the SSPP, desorption rate of drug from particulate, number of particles or size of particle in unit volume, dissolving rate of the matrix, number of SSPP perforators, size of the SSPP patch, size and composition of the reservoir, and frequency of using the device etc. Most applications of SSPP drug transdermal delivery target the epidermis, although delivery into blood stream directly is available by extending the penetration length of an SSPP patch.

The SSPP patch systems disclosed herein are also useful for controlling transport across tissues other than skin. For example, an SSPP patch can be inserted into a patient's eye to control or correct conjunctiva, sclera, and/or cornea problems, to facilitate delivery of drugs into the eye with a slow moving actuator. The drug-loaded particle stays in the tissue for sustained drug delivery even after the patch is removed. Similarly, an SSPP system, inserted into the eye, can facilitate transport of fluid out of the eye, which may be of benefit for treatment of glaucoma. An SSPP patch can also be inserted into the buccal (oral cavity, e.g., for breakthrough pain management), nasal or vaginal regions or inside a tissue with the aid of a laparoscope or into other accessible mucosal layers to facilitate transport into or across those tissues. For example, a drug may be delivered across the buccal mucosa for local treatment in the mouth or gingiva, or to act as a muscle relaxant for orthodontic applications. As another example, SSPP systems may be used internally within the body on, for example, the lining of the gastrointestinal tract to facilitate uptake of orally-ingested drugs or at the lining of blood vessels to facilitate penetration of drugs into the vessel wall. In the case of internal tissue application, use of a bioadhesive SSPP material can be an additional benefit.

Another important application is vaccination. The skin is an ideal site for effective vaccine delivery because it contains a network of immune cells, such as Langerhans cells. There are several advantages of SSPP technology with vaccine-loaded particles which can also serve as adjuvants when delivering immunogenic compounds to the epidermis. The epidermis has a high density of immune cells and consequently triggers the immune system more effectively. An SSPP system with vaccine-loaded particles can reduce loading dose and induce rapid delivery to Langerhans cell and can provide a depot effect. In a vaccine application, the particle can be an alum particle to enhance vaccine efficacy. The SSPP system can be easily designed for multivalent vaccines and is expected to provide more stability than the use of a liquid for transport and storage of drugs. The following list provides nonlimiting examples of vaccines that can be delivered using these systems.
Hepatitis A, B and C;
HIV vaccine;
Influenza;
Diphtheria;
Tetanus;
Pertussis;
Lyme disease;
Rabies;
Pneumococcus;
Yellow fever;
Cholera;
Vaccinia;
Tuberculosis;
Rubella;
Measles;
Mumps;
Rotavirus;
Botulinum;
Herpes virus;
Other DNA vaccines.

Another area of applications is cosmeceutical. An SSPP system with particles can be used efficiently and safely to remove or reduce wrinkle formation, skin aging hyperhidrosis. For example, botox toxin, hydroxyacid, vitamins and vitamin derivatives, and the like, can be delivered using the systems described herein. The systems are also useful for treating lesions or abnormal skin features, such as pimples, acne, corns, warts, calluses, bunions, actinic keratoses and hard hyperkeratotic skin, which is often found on the face, arms, legs or feet. An SSPP system is also useful as a tattoo creating patch for cosmetic application and as a food patch to deliver essential amino acids, fats and vitamins. A food patch is often used in emergencies.

## Claims

1. A method of producing a microneedle with a selected drug concentrated in the tip or on the tip surface, said method comprising:
(a) providing a particulate component selected from the group consisting of a particulate drug, and an inert particle with a drug adsorbed thereto;
(b) combining said particulate component with a soluble matrix material to form a suspension solution comprising said particulate component;
(c) casting said suspension solution into a microneedle mold;
(d) centrifuging said cast microneedle mold under conditions that move the particulate component into the microneedle tip or tip surface; and
(e) drying and separating the cast microneedle from the mold.

2. The method of claim 1, wherein the particulate component is a particulate drug.

3. The method of claim 1, wherein the particulate component is an inert particle with a drug adsorbed thereto.

4. The method of claim 3, wherein the drug is a vaccine.

5. The method of claim 4, wherein the inert particle is poly (lactic-co-glycolic acid) (PLGA) or aluminum hydroxide and aluminum phosphate (alum).

6. The method of claim 1, wherein the drug is a protein.

7. The method of claim 1, wherein the matrix material is a hydrogel.

8. The method of claim 7, wherein the matrix material comprises sodium carboxymethyl cellulose.

9. A method of producing a microneedle with a selected drug concentrated in the tip or on the tip surface, said method comprising:
(a) combining a selected drug, a soluble matrix material and an inert particle in solution to form a suspension solution comprising the inert particle with said drug and matrix adsorbed thereto;
(b) casting said suspension solution into a microneedle mold;
(c) centrifuging said cast microneedle mold under conditions that move the drug-adsorbed inert particle into the microneedle tip or surface of the microneedle; and
(d) drying and separating the cast microneedle from the mold.

10. The method of claim 9, wherein the drug is a vaccine.

11. The method of claim 10, wherein the inert particle is poly (lactic-co-glycolic acid) (PLGA) or aluminum hydroxide and aluminum phosphate (alum).

12. The method of claim 9, wherein the drug is a protein.

13. The method of claim 9, wherein the matrix material is a hydrogel.

14. The method of claim 13, wherein the matrix material comprises sodium carboxymethyl cellulose.

15. A method of producing a microneedle with a selected drug concentrated in the tip or on the tip surface, said method comprising:
(a) providing a particulate component selected from the group consisting of a dried particulate drug, and a dried inert particle with a drug adsorbed thereto;
(b) adding said particulate component into the tip portion of a microneedle mold;
(c) packing a powdered matrix onto the particulate component to fill the microneedle mold;
(d) applying a compressive force to the packed microneedle mold to solidify the microneedle; and
(e) drying and separating the cast microneedle from the mold.

16. The method of claim 15, wherein the particulate component is a particulate drug.

17. The method of claim 15, wherein the particulate component is an inert particle with a drug adsorbed thereto.

18. The method of claim 17, wherein the drug is a vaccine.

19. The method of claim 18, wherein the inert particle is poly (lactic-co-glycolic acid) (PLGA) or aluminum hydroxide and aluminum phosphate (alum).

20. The method of claim 15, wherein the drug is a protein.

21. The method of claim 15, wherein the matrix material is a hydrogel.

22. The method of claim 21, wherein the matrix material comprises sodium carboxymethyl cellulose.

## Patentansprüche

1. Verfahren zur Herstellung einer Mikronadel mit einem ausgewählten Wirkstoff, welcher in der Spitze oder der Oberfläche der Spitze angereichert ist, wobei das Verfahren aufweist:
(a) Bereitstellen einer partikelförmigen Komponente, welche aus der Gruppe, welche aus einem partikelförmigen Wirkstoff, und einem inerten Partikel mit einem Wirkstoff, welcher auf diesem absorbiert ist, besteht, ausgewählt ist;
(b) Kombinieren der partikelförmigen Komponente mit einem löslichen Matrixmaterial, um eine Suspensionslösung zu bilden, welche die partikelförmige Komponente aufweist;
(c) Gießen der Suspensionslösung in eine Mikronadelform;
(d) Zentrifugieren der Mikronadelform unter Bedingungen, bei denen die partikelförmige Komponente an die Spitze oder die Oberfläche der Spitze der Mikronadel bewegt wird; und
(e) Trocknen und Trennen der gegossenen Mikronadel von der Form.

2. Verfahren gemäß Anspruch 1, wobei die partikelförmige Komponente ein partikelförmiger Wirkstoff ist.

3. Verfahren gemäß Anspruch 1, wobei die partikelförmige Komponente ein inerter Partikel ist, auf dem ein Wirkstoff adsorbiert ist.

4. Verfahren gemäß Anspruch 3, wobei der Wirkstoff ein Impfstoff ist.

5. Verfahren gemäß Anspruch 4, wobei der inerte Partikel Poly(Milch-Co-Glycolsäure)(PLGA) oder Aluminiumhydroxid und Aluminiumphosphat (Alum) ist.

6. Verfahren gemäß Anspruch 1, wobei der Wirkstoff ein Protein ist.

7. Verfahren gemäß Anspruch 1, wobei das Matrixmaterial ein Hydrogel ist.

8. Verfahren gemäß Anspruch 7, wobei das Matrixmaterial Natriumcarboxymethylcellulose aufweist.

9. Verfahren zur Herstellung einer Mikronadel mit einem ausgewählten Wirkstoff, welcher in der Spitze oder auf der Oberfläche der Spitze angereichert ist, wobei das Verfahren aufweist:
(a) Kombinieren eines ausgewählten Wirkstoffs, eines löslichen Matrixmaterials und eines inerten Partikels in Lösung, um eine Suspensionslösung zu bilden, welche den inerten Partikel mit dem Wirkstoff und der Matrix, welche auf diesem adsorbiert sind, aufweist;
(b) Gießen der Suspensionslösung in eine Mikronadelform;
(c) Zentrifugieren der Mikronadelform unter Bedingungen, bei denen sich der inerte Partikel, auf dem ein Wirkstoff adsorbiert ist, zur Spitze oder zur Oberfläche der Spitze der Mikronadel bewegt;
und
(d) Trocknen und Trennen der gegossenen Mikronadel von der Form.

10. Verfahren gemäß Anspruch 9, wobei der Wirkstoff ein Impfstoff ist.

11. Verfahren gemäß Anspruch 10, wobei der inerte Partikel Poly(Milch-Co-Glycolsäure)(PLGA) oder Aluminiumhydroxid und Aluminiumphosphat (Alum) ist.

12. Verfahren gemäß Anspruch 9, wobei der Wirkstoff ein Protein ist.

13. Verfahren gemäß Anspruch 9, wobei das Matrixmaterial ein Hydrogel ist.

14. Verfahren gemäß Anspruch 13, wobei das Matrixmaterial Natriumcarboxymethylcellulose umfasst.

15. Verfahren zum Herstellen einer Mikronadel mit einem ausgewählten Wirkstoff, welcher in der Spitze oder der Oberfläche der Spitze angereichert ist, wobei das Verfahren aufweist:
(a) Bereitstellen einer partikelförmigen Komponente, welche aus der Gruppe, welche aus einem getrockneten partikelförmigen Wirkstoff und einem getrockneten inerten Partikel mit einem Wirkstoff, welcher auf diesem adsorbiert ist, besteht, ausgewählt ist;
(b) Hinzufügen von der partikelförmigen Komponente zum Spitzenabschnitt einer Mikronadelform;
(c) Verdichten einer pulverförmigen Matrix auf die partikelförmige Komponente, um die Mikronadelform zu füllen;
(d) Aufwenden einer Druckkraft auf die verdichtete Mikronadelform, um die Mikronadel zu verfestigen; und
(e) Trocknen und Trennen der gegossenen Mikronadel von der Form.

16. Verfahren gemäß Anspruch 15, wobei die partikelförmige Komponente ein partikelförmiger Wirkstoff ist.

17. Verfahren gemäß Anspruch 15, wobei die partikelförmige Komponente ein inerter Partikel mit einem Wirkstoff, welcher auf diesem adsorbiert ist, ist.

18. Verfahren gemäß Anspruch 17, wobei der Wirkstoff ein Impfstoff ist.

19. Verfahren gemäß Anspruch 18, wobei der inerte Partikel Poly(Milch-Co-Glycolsäure)(PLGA) oder Aluminiumhydroxid und Aluminiumphosphat (Alum) ist.

20. Verfahren gemäß Anspruch 15, wobei der Wirkstoff ein Protein ist.

21. Verfahren gemäß Anspruch 15, wobei das Matrixmaterial ein Hydrogel ist.

22. Verfahren gemäß Anspruch 21, wobei das Matrixmaterial Natriumcarboxymethylcellulose aufweist.

## Revendications

1. Procédé de production d'une micro-aiguille avec un médicament choisi concentré dans l'extrémité ou sur la surface de l'extrémité, ledit procédé comprenant :
(a) la fourniture d'un composant particulaire choisi dans le groupe constitué d'un médicament particulaire, et d'une particule inerte avec un médicament qui y est adsorbé ;
(b) la combinaison dudit composant particulaire avec un matériau de matrice soluble pour former une solution de suspension comprenant ledit composant particulaire ;
(c) le moulage de ladite solution de suspension dans un moule de micro-aiguille ;
(d) la centrifugation du moule de la micro-aiguille moulée dans des conditions qui déplacent le composant particulaire dans l'extrémité ou la surface de l'extrémité de la micro-aiguille ; et
(e) le séchage et la séparation de la micro-aiguille du moule.

2. Procédé selon la revendication 1, dans lequel le composant particulaire est un médicament particulaire.

3. Procédé selon la revendication 1, dans lequel le composant particulaire est une particule inerte avec un médicament qui y est adsorbé.

4. Procédé selon la revendication 3, dans lequel le médicament est un vaccin.

5. Procédé selon la revendication 4, dans lequel la particule inerte est du poly(acide lactique-co-glycolique) (PLGA) ou de l'hydroxyde d'aluminium et du phosphate d'aluminium (alun).

6. Procédé selon la revendication 1, dans lequel le médicament est une protéine.

7. Procédé selon la revendication 1, dans lequel le matériau de matrice est un hydrogel.

8. Procédé selon la revendication 7, dans lequel le matériau de matrice comprend de la carboxyméthylcellulose sodique.

9. Procédé de production d'une micro-aiguille avec un médicament choisi concentré dans l'extrémité ou sur la surface de l'extrémité, ledit procédé comprenant :
(a) la combinaison d'un médicament choisi, d'un matériau de matrice soluble et d'une particule inerte en solution pour former une solution de suspension comprenant la particule inerte avec lesdits médicament et matrice qui y sont adsorbés ;
(b) le moulage de ladite solution de suspension dans un moule de mïcro-aiguille ;
(c) la centrifugation dudit moule de micro-aiguille moulée dans des conditions qui déplacent la particule inerte avec le médicament adsorbé dans l'extrémité de la micro-aiguille ou la surface de la micro-aiguille ; et
(d) le séchage et la séparation de la micro-aiguille moulée du moule.

10. Procédé selon la revendication 9, dans lequel le médicament est un vaccin.

11. Procédé selon la revendication 10, dans lequel la particule inerte est du poly(acide lactique-co-glycolique) (PLGA) ou de l'hydroxyde d'aluminium et du phosphate d'aluminium (alun).

12. Procédé selon la revendication 9, dans lequel le médicament est une protéine.

13. Procédé selon la revendication 9, dans lequel le matériau de matrice est un hydrogel.

14. Procédé selon la revendication 13, dans lequel le matériau de matrice comprend de la carboxyméthylcellulose sodique.

15. Procédé de production d'une micro-aiguille avec un médicament choisi concentré dans l'extrémité ou sur la surface de l'extrémité, ledit procédé comprenant :
(a) la fourniture d'un composant particulaire choisi dans le groupe constitué d'un médicament particulaire séché, et d'une particule inerte séchée, avec un médicament qui y est adsorbé ;
(b) l'addition dudit composant particulaire dans la partie de l'extrémité d'un moule de micro-aiguille ;
(c) le tassement d'une matrice en poudre sur le composant particulaire pour remplir le moule de micro-aiguille ;
(d) l'application d'une force de compression sur le moule de micro-aiguille tassé pour solidifier la micro-aiguille ; et
(e) le séchage et la séparation de la micro-aiguille moulée du moule.

16. Procédé selon la revendication 15, dans lequel le composant particulaire est un médicament particulaire.

17. Procédé selon la revendication 15, dans lequel le composant particulaire est une particule inerte avec un médicament qui y est adsorbé.

18. Procédé selon la revendication 17, dans lequel le médicament est un vaccin.

19. Procédé selon la revendication 18, dans lequel la particule inerte est du poly(acide lactique-co-glycolique) (PLGA) ou de l'hydroxyde d'aluminium et du phosphate d'aluminium (alun).

20. Procédé selon la revendication 15, dans lequel le médicament est une protéine.

21. Procédé selon la revendication 15, dans lequel le matériau de matrice est un hydrogel.

22. Procédé selon la revendication 21, dans lequel le matériau de matrice comprend de la carboxyméthylcellulose sodique.
